# EUROPEAN PATENT SPECIFICATION

(11) **EP 1 297 011 B1**
(45) Date of publication and mention of the grant of the patent: **30.08.2006**
(21) Application number: 01960451.1
(22) Date of filing: 03.07.2001
(51) Int. Cl.: C07K 14/47, C12N 5/10, C07K 16/18, A61K 38/17, A61K 48/00, A61K 39/395, A61P 37/06, A61P 37/08, A61P 35/00, A61P 31/00

(54) **DIAGNOSTIC USES OF THE T-CELL PROTEIN TZON7, AND OF THE PEPTIDES AND ANTIBODIES DERIVED THEREFROM**
DIAGNOSTISCHE VERWENDUNGEN DES T-ZELL PROTEINS TZON7, DER DAVON ABGELEITETEN PEPTIDE UND DES ANTIKÖRPERS
UTILISATION DIAGNOSTIQUE DE LA NOUVELLE PROTEINE DE CELLULES-T (TZON7), DES PEPTIDES ET DES ANTICORPS DERIVES DE CEUX-CI

(30) Priority: 03.07.2000 EP 00114234
(43) Date of publication of application: 02.04.2003
(73) Proprietor: Utku, Nalan, 10719 Berlin (DE)
(72) Inventor: Utku, Nalan, 10719 Berlin (DE)
(74) Representative: Müller Fottner Steinecke
(86) International application number: PCT/EP2001/007610
(87) International publication number: WO 2002/002619

(56) References cited:
- WO-A-00/61614
- WO-A-00/78953
- DATABASE GENEMBL [Online] 18 April 1997 (1997-04-18) ADAMS,M.D. ET AL.: "EST183376 Jurkat T-cells VI Homo sapiens cDNA, 5' end" XP002183513
- UTKU NALAN ET AL: "The human homolog of Drosophila cornichon protein is differentially expressed in alloactivated T-cells." BIOCHIMICA ET BIOPHYSICA ACTA, vol. 1449, no. 3, 1 April 1999 (1999-04-01), pages 203-210, XP000982809 ISSN: 0006-3002

### SEQUENCE LISTING

<110> UTKU, Nalan
<120> A novel T-cell protein (TZON7), peptides and antibodies derived therefrom and uses thereof
<130> E 2036 PCT
<140>
   <141>
<150> EP 00 11 4234.8
   <151> 2000-07-03
<160> 13
<170> PatentIn Ver. 2.1
<210> 1
   <211> 135
   <212> DNA
   <213> Homo sapiens
   <220>
   <221> CDS
   <222> (3)..(134)
<400> 1
   gc ttc agc act ggg gcc tat agt tca gac agc tgt tca gcg gaa gag    47
      Phe Ser Thr Gly Ala Tyr Ser Ser Asp Ser Cys Ser Ala Glu Glu
        1               5                  10                  15
   ggt ttg cac tgc agg ccc ctc tct tgc aga agt gga ggt gag gct tgt   95
   Gly Leu His Cys Arg Pro Leu Ser Cys Arg Ser Gly Gly Glu Ala Cys
                    20                  25                  30
   ctc tgc ttc aag gac tct ctc tgc aac ctc caa cag gaa g             135
   Leu Cys Phe Lys Asp Ser Leu Cys Asn Leu Gln Gln Glu
                35                  40
<210> 2
   <211> 44
   <212> PRT
   <213> Homo sapiens
<400> 2
   Phe Ser Thr Gly Ala Tyr Ser Ser Asp Ser Cys Ser Ala Glu Glu Gly
     1               5                  10                  15
   Leu His Cys Arg Pro Leu Ser Cys Arg Ser Gly Gly Glu Ala Cys Leu
                20                  25                  30
   Cys Phe Lys Asp Ser Leu Cys Asn Leu Gln Gln Glu
            35                  40
<210> 3
   <211> 135
   <212> DNA
   <213> Homo sapiens
<400> 3
   gcttcagcac tggggcctat agttcagaca gctgttcagc ggaagagggt ttgcactgca 60
   ggcccctctc ttgcagaagt ggaggtgagg cttgtctctg cttcaaggac tctctctgca 120
   acctccaaca ggaag                                                  135
<210> 4
   <211> 43
   <212> PRT
   <213> Homo sapiens
<400> 4
   Phe Ser Thr Gly Ala Tyr Ser Ser Asp Ser Cys Ser Ala Glu Glu Gly
   1               5                   10                  15
   Leu His Cys Arg Pro Leu Ser Cys Arg Ser Gly Gly Glu Ala Cys Leu
               20                  25                  30
   Cys Phe Lys Asp Ser Leu Cys Leu Gln Gln Glu
           35                  40
<210> 5
   <211> 363
   <212> PRT
   <213> Homo sapiens
<400> 5
   Gly Arg Asn Lys Met Asp Pro Pro Arg Ser Ser Ile Phe Leu Gln Glu
   1               5                   10                  15
   Val Ile Thr Thr Val Tyr Gly Tyr Lys Val Gln Leu Gln Ala Gly Leu
               20                  25                  30
   Glu Leu Val Val Asn Asn Gln Lys Met Ala Val Pro Tyr Arg Pro Asn
           35                  40                  45
   Glu His Leu Arg Val Thr Leu Arg Gly Gln Arg Leu Tyr Leu Val Thr
       50                  55                  60
   Asp Phe Glu Leu Val Val Ser Phe Gly Gly Arg Lys Asn Ala Val Ile
   65                  70                  75                  80
   Ser Leu Pro Ser Met Tyr Glu Gly Leu Val Ser Gly Leu Cys Gly Asn
                   85                  90                  95
   Tyr Asp Lys Asn Arg Lys Asn Asp Met Met Leu Pro Ser Gly Ala Leu
               100                 105                 110
   Thr Gln Asn Leu Asn Thr Phe Gly Asn Ser Trp Glu Val Lys Thr Glu
           115                 120                 125
   Asp Ala Leu Leu Arg Phe Pro Arg Ala Ile Pro Ala Glu Glu Glu Gly
       130                 135                 140
   Gln Gly Ala Glu Leu Gly Leu Arg Thr Gly Leu Gln Val Ser Glu Cys
   145                 150                 155                 160
   Ser Pro Glu Gln Leu Ala Ser Asn Ser Thr Gln Ala Cys Arg Val Leu
                   165                 170                 175
   Ala Asp Pro Gln Ala Pro Phe Ala Ala Cys His Gln Thr Val Pro Pro
               180                 185                 190
   Glu Pro Phe Gln Glu His Cys Val Leu Asp Leu Cys Ser Ala Gln Asp
           195                 200                 205
   Pro Arg Glu Gln Glu Glu Leu Arg Cys Gln Val Leu Ser Gly Tyr Ala
       210                 215                 220
   Ile Leu Cys Gln Glu Ala Gly Ala Ala Leu Ala Gly Trp Arg Asp Arg
   225                 230                 235                 240
   Thr Leu Cys Ala Met Glu Cys Pro Ala Gly Thr Ile Tyr Gln Ser Cys
                   245                 250                 255
   Met Thr Pro Cys Pro Ala Ser Cys Ala Asn Leu Ala Asp Pro Gly Asp
               260                 265                 270
   Cys Glu Gly Pro Cys Val Glu Gly Cys Ala Asp Pro Arg Leu Cys Leu
           275                 280                 285
   Gln Trp His Pro Glu Pro Pro Leu Ala Asp Cys Gly Cys Thr Ser Asn
       290                 295                 300
   Gly Ile Tyr Tyr Gln Val Arg Ala Gly Ser Arg Arg Pro Trp Gly Ala
   305                 310                 315                 320
   Glu Ala Pro Arg Arg Ala Arg Pro Gly Met Glu Leu Glu Arg Leu Leu
                   325                 330                 335
   Leu Ala Leu Pro Phe Leu Ala Gly Gln Gln Phe Leu Thr Glu Asp Cys
               340                 345                 350
   Ser Gln Arg Cys Thr Cys Ala Ser Ser Arg Ile
           355                 360
<210> 6
   <211> 15
   <212> DNA
   <213> Artificial Sequence
<220>
   <223> Description of Artificial Sequence: artificial
<400> 6
   1H tgcttcagca ctgcc 15 1H
<210> 7
   <211> 18
   <212> DNA
   <213> Artificial Sequence
<220>
   <223> Description of Artificial Sequence: artificial
<400> 7
   1H ttattgtatt tgaagtaa 18 1H
<210> 8
<211> 825
   <212> DNA
   <213> Homo sapiens
<220>
   <221> CDS
   <222> (1)..(822)
<400> 8
   atg gac cgg ccg ggg ttc gtg gca gcg ctg gtg gct ggt ggg gta gca 48
   Met Asp Arg Pro Gly Phe Val Ala Ala Leu Val Ala Gly Gly Val Ala
   1 5 10 15
   ggt gtt tct gtt gac ttg ata tta ttt cct ctg gat acc att aaa acc 96
   Gly Val Ser Val Asp Leu Ile Leu Phe Pro Leu Asp Thr Ile Lys Thr
   20 25 30
   agg ctg cag agt ccc caa gga ttt aat aag gct ggt ggt ttt cat gga 144
   Arg Leu Gln Ser Pro Gln Gly Phe Asn Lys Ala Gly Gly Phe His Gly
   35 40 45
   ata tat gct ggc gtt cct tct gct gct att gga tcc ttt cct aat gct 192
   Ile Tyr Ala Gly Val Pro Ser Ala Ala Ile Gly Ser Phe Pro Asn Ala
   50 55 60
   gct gca ttt ttt atc acc tat gaa tat gtg aag tgg ttt ttg cat gct 240
   Ala Ala Phe Phe Ile Thr Tyr Glu Tyr Val Lys Trp Phe Leu His Ala
   65 70 75 80
   gat tca tct tca tat ttg aca cct atg aaa cat atg ttg gct gcc tct 288
   Asp Ser Ser Ser Tyr Leu Thr Pro Met Lys His Met Leu Ala Ala Ser
   85 90 95
   gct gga gaa gtg gtt gcc tgc ctg att cga gtt cca tct gaa gtg gtt 336
   Ala Gly Glu Val Val Ala Cys Leu Ile Arg Val Pro Ser Glu Val Val
   100 105 110
   aag cag agg gca cag gta tct gct tct aca aga aca ttt cag att ttc 384
   Lys Gln Arg Ala Gln Val Ser Ala Ser Thr Arg Thr Phe Gln Ile Phe
   115 120 125
   tct aac atc tta tat gaa gag ggt atc caa ggg ttg tat cga ggc tat 432
   Ser Asn Ile Leu Tyr Glu Glu Gly Ile Gln Gly Leu Tyr Arg Gly Tyr
   130 135 140
   aaa agc aca gtt tta aga gag att cct ttt tct ttg gtc cag ttt ccc 480
   Lys Ser Thr Val Leu Arg Glu Ile Pro Phe Ser Leu Val Gln Phe Pro
   145 150 155 160
   tta tgg gag tcc tta aaa gcc ctc tgg tcc tgg agg cag gat cat gtg 528
   Leu Trp Glu Ser Leu Lys Ala Leu Trp Ser Trp Arg Gln Asp His Val
   165 170 175
   gtg gat tct tgg cag tca gca gtc tgt gga gct ttt gca ggt gga ttt 576
   Val Asp Ser Trp Gln Ser Ala Val Cys Gly Ala Phe Ala Gly Gly Phe
   180 185 190
   gcc gct gca gtc acc acc cct cta gac gtg gca aag aca aga att acg 624
   Ala Ala Ala Val Thr Thr Pro Leu Asp Val Ala Lys Thr Arg Ile Thr
   195 200 205
   ctg gca aag gct ggc tcc agc act gct gat ggg tat gtg ctc tct gtc 672
   Leu Ala Lys Ala Gly Ser Ser Thr Ala Asp Gly Tyr Val Leu Ser Val
               210                 215                 220
   ctg cat ggg gtc tgg cgg tca cag ggg ctg gca gga tta ttt gca ggt      720
   Leu His Gly Val Trp Arg Ser Gln Gly Leu Ala Gly Leu Phe Ala Gly
   225                     230                 235                 240
   gtc ttc cct cga atg gca gcc atc agt ctg gga ggt ttc atc ttt ctg      768
   Val Phe Pro Arg Met Ala Ala Ile Ser Leu Gly Gly Phe Ile Phe Leu
                       245                 250                 255
   ggg gct tat gac cga acg cac agc ttg ctg ttg gaa gtt ggc aga aag      816
   Gly Ala Tyr Asp Arg Thr His Ser Leu Leu Leu Glu Val Gly Arg Lys
                   260                 265                 270
   agt cct tga                                                          825
   Ser Pro
<210> 9
   <211> 274
   <212> PRT
   <213> Homo sapiens
<400> 9
   Met Asp Arg Pro Gly Phe Val Ala Ala Leu Val Ala Gly Gly Val Ala
    1               5                  10                  15
   Gly Val Ser Val Asp Leu Ile Leu Phe Pro Leu Asp Thr Ile Lys Thr
               20                  25                  30
   Arg Leu Gln Ser Pro Gln Gly Phe Asn Lys Ala Gly Gly Phe His Gly
           35                  40                  45
   Ile Tyr Ala Gly Val Pro Ser Ala Ala Ile Gly Ser Phe Pro Asn Ala
       50                  55                  60
   Ala Ala Phe Phe Ile Thr Tyr Glu Tyr Val Lys Trp Phe Leu His Ala
   65                  70                  75                  80
   Asp Ser Ser Ser Tyr Leu Thr Pro Met Lys His Met Leu Ala Ala Ser
                   85                  90                  95
   Ala Gly Glu Val Val Ala Cys Leu Ile Arg Val Pro Ser Glu Val Val
               100                 105                 110
   Lys Gln Arg Ala Gln Val Ser Ala Ser Thr Arg Thr Phe Gln Ile Phe
           115                 120                 125
   Ser Asn Ile Leu Tyr Glu Glu Gly Ile Gln Gly Leu Tyr Arg Gly Tyr
       130                 135                 140
   Lys Ser Thr Val Leu Arg Glu Ile Pro Phe Ser Leu Val Gln Phe Pro
   145                 150                 155                 160
   Leu Trp Glu Ser Leu Lys Ala Leu Trp Ser Trp Arg Gln Asp His Val
                   165                 170                 175
   Val Asp Ser Trp Gln Ser Ala Val Cys Gly Ala Phe Ala Gly Gly Phe
               180                 185                 190
   Ala Ala Ala Val Thr Thr Pro Leu Asp Val Ala Lys Thr Arg Ile Thr
           195                 200                 205
   Leu Ala Lys Ala Gly Ser Ser Thr Ala Asp Gly Tyr Val Leu Ser Val
       210                 215                 220
   Leu His Gly Val Trp Arg Ser Gln Gly Leu Ala Gly Leu Phe Ala Gly
   225                 230                 235                 240
   Val Phe Pro Arg Met Ala Ala Ile Ser Leu Gly Gly Phe Ile Phe Leu
                   245                 250                 255
   Gly Ala Tyr Asp Arg Thr His Ser Leu Leu Leu Glu Val Gly Arg Lys
               260                 265                 270
   Ser Pro
<210> 10
   <211> 14
   <212> PRT
   <213> Artificial Sequence
<220>
   <223> Description of Artificial Sequence: artificial
<400> 10
   Lys Thr Arg Leu Gln Ser Pro Gln Gly Phe Asn Lys Ala Gly
    1               5                   10
<210> 11
   <211> 11
   <212> PRT
   <213> Artificial Sequence
   <220>
   <223> Description of Artificial Sequence: artificial
<400> 11
   Gly Val Pro Ser Ala Ala Ile Gly Ser Phe Pro
    1               5                   10
<210> 12
   <211> 12
   <212> PRT
   <213> Artificial Sequence
<220>
   <223> Description of Artificial Sequence: artificial
<400> 12
   Ser Glu Val Val Lys Gln Arg Ala Gln Val Ser Ala
    1               5                   10
<210> 13
   <211> 39
   <212> PRT
   <213> Artificial Sequence
<220>
   <223> Description of Artificial Sequence: artificial
<400> 13
   Val Cys Gly Ala Phe Ala Gly Gly Phe Ala Ala Ala Val Thr Thr Pro
    1               5                   10                  15
   Leu Asp Val Ala Lys Thr Arg Ile Thr Leu Ala Lys Ala Gly Ser Ser
               20                  25                  30
   Thr Ala Asp Gly Tyr Val Leu
           35

## Description

### Field of the invention

The present invention relates to diagnostic compositions and for methods of diagnosing biological conditions employing a polynucleotide encoding a TZON7 polypeptide comprising an amino acid sequence as disclosed herein, the expression of which is upregulated during the early stages of leukocyte/lymphocyte activation in response to alloantigens. Furthermore, the application discloses a nucleic acid molecule of at least 15 nucleotides in length hybridizing specifically with a polynucleotide described herein or with a complementary strand thereof. In addition, the present invention pertains to vectors comprising polynucleotides encoding said TZON7 protein/polypeptide, (host) cells which comprise said polynucleotide(s) or said vectors, a TZON7 protein/polypeptide, an antibody which specifically recognizes a TZON7 protein or a fragment thereof, or an antisense construct capable of inhibiting the expression of a polynucleotide encoding a TZON7 protein. Finally, the present invention relates to the use of the before described polynucleotide(s), vector(s), protein(s), antisense construct(s) for the preparation of compositions for diagnosing acute and chronic diseases, involving T-cell activation and Th1, and Th2 immune response.

### Background of the invention

Immune activation is accompanied with sequential changes in the expression of various genes over several days and involves multiple signaling pathways [1]. Stimulation of T-cells is initiated by the interaction of antigen-specific T-cell receptors (TCR) with MHC bound antigenic peptides present on the surface of antigen presenting cells (APC), but full proliferative T-cell response requires additional costimulatory signals which are provided by the interaction of proteins expressed on the surface of T-cells and APC [2,3,4,5]. In addition, a number of cytokines as well as other proteins are known to augment immune activation, although many of them appear not to be essential for the basic proliferative T-cell response [3,6]. Moreover, a growing body of evidence indicates that the microtubule cytoskeleton of lymphocytes plays a major role in T-cell activation. Stimulation of T-cells was demonstrated to result in molecular rearrangement in the actin cytoskeleton leading to re-localization and concentration of signaling molecules in restricted areas of the cell membrane close to the bound APC [7,8,9].

Although considerable information on T-cell activation has been gathered in recent years, the complex molecular mechanisms of stimulation and signaling pathways are not completely understood. Since T-cell activation provides the central event in various types of inflammation as well as in autoimmune diseases and graft rejection, knowledge about the distinct steps and molecules involved in the stimulation process is of considerable biomedical importance, as they might provide targets for therapeutic modulation of the immune response. Therapeutic prevention of T-cell activation in organ transplantation and autoimmune diseases presently relies on panimmunosuppressive drugs interfering with downstream intracellular events.

Alloreactive CD4 or CD8 cells or specific alloantibodies are capable of mediating, inter alia, allograft rejection. The following immune mechanisms cause graft rejection by different mechanisms:
(a) alloactive T-cells can recruit and activate macrophages, initiating graft injury by "delayed-type" hypersensitivity response;
(b) alloactive cytotoxic T-cells are capable of directly lysing graft endothelial and parenchymal cells; and
(c) alloantibodies bind to endothelium, activate the complement system, and injure thereby graft blood vessels.

The various forms of (allograft) rejection imply a temporal sequence of events including hyperacute, acute vascular and acute cellular as well as chronic rejection.

Hyperacute rejection plays an important role in xenotransplantation and is due to natural antibodies (ref: Milford, E., Utku, A, N. Guidelines for use of immunogenetic tests organ transplantation, Manual of Clinical Laboratory Immunology, ASM Press 1997). Hyperacute rejection is mediated by preexisting antibodies that bind to endothelium and activate complement and is characterized by rapid thrombotic occlusion of the graft vasculature. In more recent clinical experience, hyperacute rejection of allografts is usually mediated by antibodies directed against protein alloantigens, such as foreign MHC molecules, or against less well described alloantigens expressed on vascular endothelial cells. Such antibodies generally arise as a result of prior exposure to alloantigens through blood transfusion, prior transplantation, or multiple pregnancies (loc. cit.). These antibodies are often of the IgG type. By testing recipients for the presence of such reactive antibodies with the cells potential donors, hyperacute rejection has been virtually eliminated from clinical allo-transplantation but remains a major problem in xenotransplantation.

Acute vascular rejection is mediated by IgG antibodies produced by B-cells against endothelial alloantigens and involves activation of complement. T-cells contribute to vascular injury by responding to alloantigens present on endothelial cells, leading to direct cell lysis of these cells, or the production of cytokines that recruit and activate inflammatory cells.

Acute cellular rejections is characterized by parenchymal cells and is usually accompanied by lymphocyte and macrophage infiltrates. These infiltrating leucocytes are responsible for the lysis of the graft. Several different effector mechanism may be involved in acute cellular rejection including CTL-mediated lysis, activated-macrophage-mediated lysis (as delayed type hypersensibility, DTH) and natural killer cells mediated lysis.

The identification of both antibody and lymphocytes as important effector mechanism in acute graft rejection suggests that this process is similar to normal antiviral immune responses. The basis of similarity probably arises from the fact that the foreign class I MHC molecules present in the graft are recognized as if they were self MHC molecules associated with endogenously synthesized foreign peptides.

Chronic rejection is characterized by fibrosis with loss of normal organ structures. The fibrosis may represent wound healing following the cellular necrosis of acute rejection or a form of DTH in which activated macrophages secrete mesenchymal cell growth factors, or alternatively chronic rejection is a response to chronic ischemia caused by injury of blood vessels. Vascular occlusion is due to proliferation of intimal smooth muscle cells, called accelerated or graft arteriosclerosis. This feature of chronic inflammation is characterized by fibrosis in autoimmune diseases such as lupus erythrematodes, sklerodermia and panarteritis nodosa. Chronic inflammatory immune response involves all parts of cellular and humoral immune system (T-, B-, NK- cells, monocytes) and is the response to the extensive production of autoantibodies and creation of immune complexes against different cellular components. This leads to multiple organ failure caused by significant tissues damage such as myositis, polyneuropathia, heart disease, vasculitis, etc.

Considering, inter alia, the above described temporal sequence events in allograft and xenograft rejection, it is desired to specifically modulate lymphocyte/leukocyte cell responses, i.e. to modulate T-, B-, NK-cells and/or monocyte responses during immunological processes. Furthermore, it is desired to specifically modulate immunological processes like, inter alia, autoimmunological events.

Specific modulation of the immune response remains, therefore, a longstanding goal in immunological research.

### Summary of the invention

The present invention relates to diagnostic uses of polynucleotides encoding an immune response modulating protein TZON7, Furthermore, the present invention relates to peptides and polypeptides derived therefrom as well as to antibodies for diagnostic uses. The uses of the invention are useful diagnostically in situations where it is desirable to modulate (antigen-specific) immune responses, e.g., inducing and maintain (antigen-specific) T-cell or B-cell non/unresponsiveness, wherein said non/unresponsiveness comprises the selective inhibition of immune cell subsets which are able to creating a response to specific antigen(s), inter alia, antigen(s) in transplanted tissue.

### Detailed description of the present invention

In view of the need of therapeutic means for the diagnosis and treatment of diseases rotated to immune responses of the human body, the technical problem of the invention is to provide means and methods for the modulation of immune cell responses which are particularly useful in organ transplantation and autoimmune diseases.
The solution to this technical problem is achieved by providing the embodiments characterized in the claims, namely a novel immune response modulating protein encoded by a cell immune response cDNA designated "TZON7" which comprises an amino acid sequence as depicted in SEQ ID NO: 2 and which exhibits a central role in leukocyte/lymphocyte activation and growth, wherein said leukocyte/lymphocyte activation refers to the activation of T-, B-, NK-cells and/or monocytes. TZON7 mRNA is transiently upregulated in the early phase of leukocyte/lymphocyte activation and in particular in T-cell activation.

In a first set of experiments, the TZON7 protein encoding cDNA has been cloned and characterized; see Example 1. Furthermore, the expression pattern of TZON7 was investigated after allo-stimulation of human leukocytes at time points 0 and 12 h and results obtained with alloactivated T-cells revealed an upregulation of TZON7 24h after immune activation of the TZON7 gene. It is thus an excellent marker for diagnosis of the status of immune response in a subject In addition, the full-length cDNA of TZON7 has been cloned, see Example 2.
The latter described results obtained in accordance with the present invention provide evidence for an essential role of TZON7 in the early events of leukocyte activation. Thus, targeting of TZON7 protein and its encoding gene provides a novel therapeutic approach for modulation of the immune response.

Accordingly, the invention relates to diagnostic uses of a polynucleotide encoding a TZON7 polypeptide, the expression of which is upregulated during the early stages of T-cell leukocyte/lymphocyte activation in response to alloantigens or a biologically active fragment thereof comprising a nucleic acid sequence selected from the group consisting of:
(I) DNA sequences encoding the amino acid sequence depicted in any one of SEQ ID NOS:9 to 13;
(II) the DNA sequence depicted in SEQ ID NO:8;
(III) DNA sequences the complementary strand of which hybridizes with and which is at least 90% identical to the polynucleotide as defined in (I) or (II); and
(IV) DNA sequences the nucleotide of which is degenerate to the nucleotide sequence of a DNA sequence of any one of (1) to ;

The term "TZON7 protein", in accordance with the present invention, denotes a protein involved in the signal transduction of leukocyteaymphocyte activation and/or proliferation and down-regulation which results in suppressing leukocyte/lymphocyte, preferably T-, B-, NK-cell and/or monocyte proliferation in response to alloactivation in a mixed lymphocyte culture when exogeneously added to the culture. In accordance with this invention it has been surprisingly found that a cDNA is differentially expressed in alloactivated lymphocytes, i.e. human T-cells. This differentially expressed cDNA was termed TZON7 and initially it was shown that this cDNA encodes for a protein sequence which is 18% homology to zonadhesin (GenBank accession number: U83191)

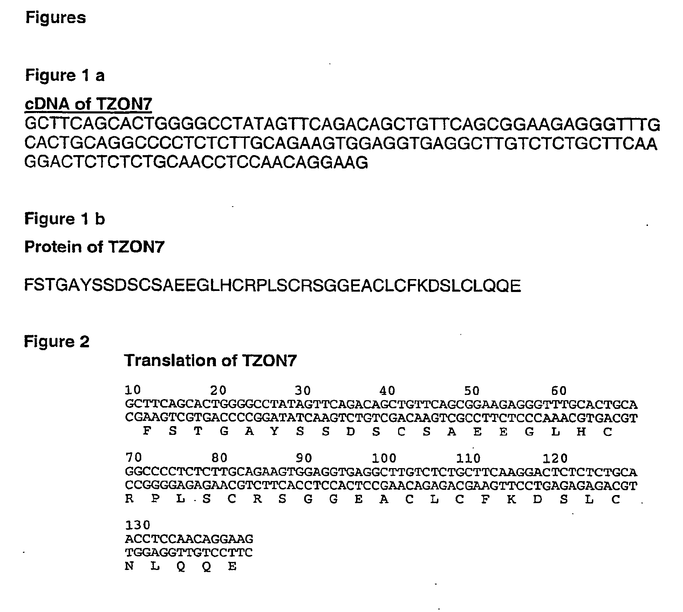

Here, it has surprisingly been found that said TZON7 protein/polypeptide plays an important role in the differentiation of quiescent T-cells to activate T-cells after alloantigen stimulation and/or cell activation/proliferation processes of B-cells, NK-cells and/or monocytes after stimulation by said allo/autoantigens or xenoantigens or by antigens from, inter alia, pathological agents, like viruses (viral agents), bacteria, etc. The term "TZON7" denotes proteins/polypeptides, in accordance with this invention, which are identical to the TZON7 protein/polypeptide as described herein (see SEQ ID NO: 9 and Figure 2) and the term comprises, furthermore, functional homologues of said protein/polypeptide such as peptides comprising an amino acid sequence as depicted in any one of SEQ ID NOS: 10 to 13. It can be concluded that TZON7 molecule is directly involved in the initiating of the immune response and might be an important target molecule for modulating the immune response.
The term "leukocyte/lymphocyte" generally denotes all kinds of white blood cells and preferably refers to monocytes and lymphocytes (B- T and NK cells), either in combination or individually. Thus, it should be understood that the term leukocyte may also be used herein so as to refer to individual species of leukocytes such as T-cells only.

In accordance with this invention the term "TZON7 polypeptide comprising an amino acid sequence as depicted in SEQ ID NO: 9" denotes a polypeptide comprising exactly said specific sequence.

The above described polynucleotide/nucleic acid molecule encoding a TZON7 polypeptide as defined herein above also comprise polynucleotides/nucleic acid molecules which encode "variants" of said TZON7 polypeptide. Such "variants" refer to polynucleotides (and/or (poly)peptides) differing from the polynucleotides and/or (poly)peptides of the invention, but retain essential properties thereof, as, inter alia, is upregulation during early stages of leukocyte/lymphocyte activation in response to xenoantigens. It is preferred that said variants are overall (closely) similar, and, preferably, in some regions identical to the polynucleotides and (poly)peptides described herein. The term "variant" in accordance with this invention comprises, but is not limited to allelic variants, synthetically produced variants or genetically engineered variants.

In accordance with the present invention, a gene induced in the early stage of T-cell activation has been identified by examining mRNA expression in alloactivated human lymphocytes. Differential display-reverse transcription PCR analysis revealed a 135bp cDNA fragment which was upregulated 24h after allostimulation of a human T-cell line; see Example 1. The corresponding (complete) cDNA named TZON7 (comprising 135 bp / predicted 44 amino acids). The deduced stretch protein shares 18% homology with human zonadhesin molecule. TZON7 is expected to function in cell proliferation and differentiation events during T-cell and/or general leukocyte activation.

Furthermore, the DNA sequence depicted in SEQ ID NO. 1 (the 135 base pair fragment AB6+m13f described in Example 1) was used to identify and clone a corresponding full-length cDNA encoding TZON7 polypeptide from cDNA libraries by conventional means and methods; see Example 2. A full length cDNA was obtained and sequence analysis revealed an 822 base pair cDNA (SEQ ID NO. 8) predicting a protein length of 274 amino acids (SEQ ID NO. 9) and thus a protein of a predicted molecular weight of about 29428.02 Dalton. Accordingly, in a preferred embodiment the polynucleotide of the invention encodes TZON7 polypeptide of about 29.4 ± 1 kDa in its non-glycosylated form. Furthermore, its estimated pl is about 9.49. Its amino acid composition is given in Example 2.

The translated aminoacid sequence of 274 amino acids does share very high hologies (>80% identities) with murine proteins AK015954 and AK015299. Beyond this, homologies has been found to be present in "Mitochondrial Carrier Protein Family, Pet8p' in *S. cerevisiae* (43% identities).

The function of the murine proteins is so far unknown. The proteins of the Pet8p family play an essential role in cellular energy metabolism. Given the sequence homology between TZON7 and "Mitochondrial Carrier Protein family Pet8p", it is likely that TZON7 could be important for growth of activated T cells and possible applied as a potential therapeutic marker or may provide novel approaches for monitoring human organ transplantation and leukemia. Means and methods to test the biological activity of the (poly)peptides of the invention, antibodies, antisense constructs and other compounds described in the general description of the invention can be performed as described in the examples of, for example, WO99/11782 and WO01/32614 which herewith are incorporated by reference.

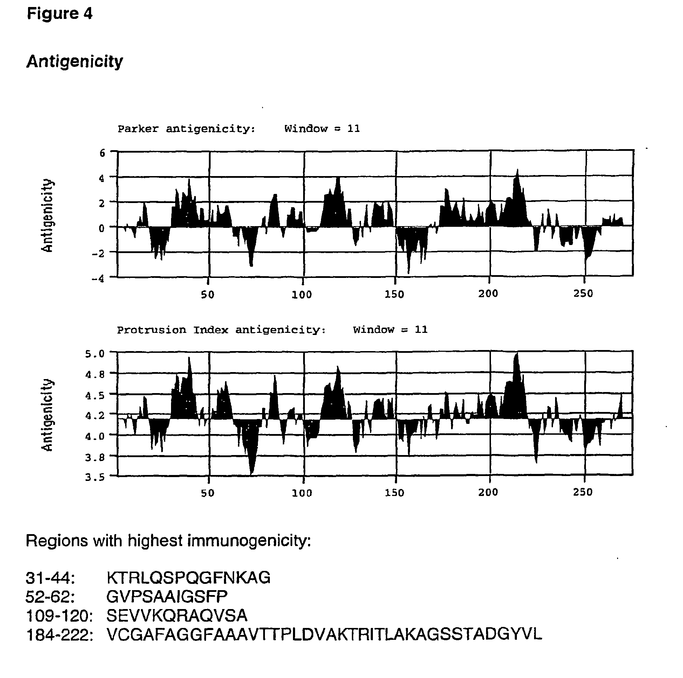

Furthermore, antigenicity analysis revealed at least four regions with highest immunogenicity; see Figure 4 and SEQ ID NOs 10 to 13. Peptides comprising anyone of these immunologically active amino acid sequences are expected to be particular suitable for, e.g., raising antibodies and/or induction of immunological responses and T-cell modulation. Accordingly, in another preferred embodiment the polynucleotide of the present invention encodes a TZON7 (poly)peptide comprising one or more of the amino acid sequences depicted in SEQ ID NOs 10 to 13. Preferably, said (poly)peptides are about 15 to 100 amino acids in length, more preferably 15 to 50, and most preferably 15 to 40 amino acids. Likewise, the present invention relates to polynucleotides encoding a polypeptide that is recognized by polyclonal and/or monoclonal antibodies raised against a peptide comprising anyone of the above-identified immunogenic regions. For example, the polynuleotide or polypeptide of the present invention can be identified by first raising antibodies against the peptide comprising, e.g., the amino acid sequence depicted in SEQ ID NO. 10 according to conventional methods and then testing a protein in question or a sample comprising such protein on a dot or Western blot with said antibodies. In case, a signal is determined due to the binding of the antibody to the protein in question, the polypeptide, or optionally its corresponding encoding DNA can be identified. Furthermore, preferably monoclonal antibodies raised against the mentioned peptides can be used to identify and isolate TZON7 proteins or fragments thereof by, for example, immunochromatography. All these methods are well within the skill of the person skilled in the art.

From the above it is evident that the nucleotide sequences depicted in SEQ ID NO: 8 encode a novel immune response modulating protein. By the provision of these nucleotide sequences it is now possible to isolate identical or similar polynucleotides which code for proteins with the biological, immunological activity of TZON7 from other species or organisms. Said nucleotide sequences may be employed, in accordance with this invention, in the pharmaceutical compositions, uses and/or methods described herein. Well-established approaches for the identification and isolation of such related sequences are, for example, the isolation from genomic or cDNA libraries using the complete or part of the disclosed sequence as a probe or the amplification of corresponding polynucleotides by polymerase chain reaction using specific primers.
Thus, the invention also relates to the use for diagnostic purposes of polynucleotides which hybridize to the above described polynucleotides and differ at one or more positions in comparison to these as long as they encode a TZON7 protein as defined above. Such molecules comprise those which are changed, for example, by deletion(s), insertion(s), alteration(s) or any other modification known in the art in comparison to the above described polynucleotides either alone or in combination. Methods for introducing such modifications in the polynucleotides of the invention are well-known to the person skilled in the art; see, e.g., Sambrook et al. (Molecular cloning; A Laboratory Manual, Second Edition, Cold Spring Harbor Laboratory Press; Cold Spring Harbor NY (1989)). The invention also relates to polynucleotides the nucleotide Sequence of which differs from the nucleotide sequence of any of the above-described polynucleotides due to the degeneracy of the genetic code.

With respect to the DNA sequences characterized under (III) above, the term "hybridizing" in this context is understood as referring to conventional hybridization conditions, preferably such as hybridization in 50%formamide/6xSSC/0.1%SDS/100µg/ml ssDNA, in which temperatures for hybridization are above 37°C and temperatures for washing in 0.1xSSC/0.1%SDS are above 55°C. Most preferably, the term "hybridizing" refers to stringent hybridization conditions, for example such as described in Sambrook, supra.

Particularly preferred are polynucleotides which preferably 90-95%, and most preferably 96-99% sequence identity with one of the above-mentioned polynucleotides and have the same biological activity. Such polynucleotides also comprise those which are altered, for example by nucleotide deletion(s), insertion(s), substitution(s), addition(s), and/or recombination(s) and/or modification(s) known in the art either alone or in combination in comparison to the above-described polynucleotides. Methods for introducing such modifications in the nucleotide sequence of the polynucleotide of the invention are well known to the person skilled in the art. Thus, the pharmaceutical composition(s), use(s) and method(s) of the present invention may comprise any polynucleotide that can be derived from the above described polynucleotides by way of genetic engineering and that encode upon expression a TZON7 protein or a biologically active fragment thereof.

It is also immediately evident to the person skilled in the art that regulatory sequences may be added to the polynucleotide as defined herein and employed in the pharmaceutical composition, uses and/or methods of the invention. For example, promoters, transcriptional enhancers and/or sequences which allow for induced expression of the polynucleotide of the invention may be employed. A suitable inducible system is for example tetracycline-regulated gene expression as described, e.g., by Gossen and Bujard (Proc. Natl. Acad. Sci. USA 89 (1992), 5547-5551) and Gossen et al. (Trends Biotech. 12 (1994), 58-62).

In a preferred embodiment the polynucleotide of the invention encodes a polypeptide comprising an amino acid sequence as depicted in SEQ ID NO:2 or 9 or a biologically active fragment thereof.

In a preferred embodiment said nucleic acid molecules are labeled. Said labels may comprise radiolabels or fluorescence labels. In another preferred embodiment said nucleic acid molecules may be used for the suppression of TZON7 expression. Particularly preferred in this embodiment are the above described hybridizing nucleic acid molecules.

The polynucleotide as employed in accordance with this invention and encoding the above described TZON7 protein or (a) biologically active fragment(s) thereof may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions. Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the P_{L}, lac, trp or tac promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40.enhancer or a globin intron in mammalian and other animal cells. Beside elements which are responsible for the initiation of transcription such regulatory elements may also comprise transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-vitrogene), or pSPORT1 (GIBCO BRL). Preferably, the expression control sequences will be eukaryotic promoter systems In vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the protein of the invention may follow; see, e.g., the appended examples. In one preferred embodiment of the present invention antisense constructs are made based on the polynucleotide encoding TZON7 (or (a) biologically active fragment(s) thereof) and combined with an appropriate expression control sequence.

In this context, it should be mentioned that a method for the production of a TZON7 protein may comprise:
(a) culturing a host described herein above under conditions allowing for the expression of the protein; or
(b) in vitro translation of the polynucleotide encoding TZON7; and recovering the protein (or a fragment thereof) produced in (a) or (b).

The transformed hosts can be grown in fermentors and cultured according to techniques known in the art to achieve optimal cell growth. The TZON7 protein and/or biological active fragments thereof can then be isolated from the growth medium, cellular lysates, or cellular membrane fractions. Once expressed, the protein of the present invention can be purified according to standard procedures of the art, including ammonium sulfate precipitation, affinity columns, column chromatography, gel electrophoresis and the like; see, Scopes, "ProteinPurification", Springer-Verlag, N.Y. (1982). Substantially pure proteins of at least about 90 to 95% homogeneity are preferred, and 98 to 99% or more homogeneity are most preferred, for pharmaceutical uses. Once purified, partially or to homogeneity as desired, the proteins may then be used therapeutically (including extracorporeally) or in developing and performing assay procedures.

Furthermore, the provision of the TZON7 protein as described herein above enables the production of TZON7 specific antibodies. In this respect, hybridoma technology enables production of cell lines secreting antibodies to essentially any desired substance that produces an immune response. RNA encoding the light and heavy chains of the immunoglobulin can then be obtained from the cytoplasm of the hybridoma. The 5' end portion of the mRNA can be used to prepare cDNA to be inserted into an expression vector. The DNA encoding the antibody or is immunoglobulin chains can subsequently be expressed in cells, preferably mammalian cells.
Depending on the host cell, renaturation techniques may be required to attain proper conformation of the antibody. If necessary, point substitutions seeking to optimize binding may be made in the DNA using conventional cassette mutagenesis or other protein engineering methodology such as is disclosed herein.

Thus, the present invention also provides the use for diagnostic purposes of an antibody specifically recognizing TZON7 protein or (a) fragment(s) (peptides, polypeptides) thereof. In a preferred embodiment of the invention, said antibody is a monoclonal antibody, a single chain antibody, humanized antibody, or fragment thereof that specifically binds said peptide or polypeptide also including bispecific antibody, synthetic antibody, antibody fragment, such as Fab, Fv or scFv fragments etc., or a chemically modified derivate of any of these. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications development by the art. Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988. When derivates of said antibodies are obtained by the phage display technique, surface plasmon resonance as employed in the BIAcore system can be used to increase the efficiency of phage antibodies which bind to an epitope of the peptide or polypeptide of the invention (Schier, Human Antibodies Hybridomas 7 (1996), 97- 105; Malmborg, J. Immunol. Methods 183 (1995), 7-13). The production of chimeric antibodies is described, for example, in WO89/09622. Methods for the production of humanized antibodies are described in, e.g., EP-A1 0 239 400 and WO90/07861. A further source of antibodies to be utilized in accordance with the present invention are so-called xenogenic antibodies. The general principle for the production of xenogenic antibodies such as human antibodies in mice is described in,e.g., WO91/10741, WO94/02602, WO96/34096 and WO96/33735. Antibodies to be employed in accordance with the invention or their corresponding immunoglobulin chain(s) can be further modified using conventional techniques known in the art, for example, by using amino acid deletion(s), insertion(s), substitution(s), addition(s), and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y. It is particularly preferred that the here described

In another embodiment the present invention relates to a diagnostic composition comprising any one of the above described proteins, antibodies, (poly)peptides, polynucleotides, vectors or cells, and optionally suitable means for detection. The (poly)peptides and antibodies described above are, for example, suited for use in immunoassays in which they can be utilized in liquid phase or bound to a solid phase carrier. Examples of immunoassays in either a direct or indirect format. Examples of such immunoassays are the radioimmunoassay (RIA), the sandwich (immunometric assay) and the Western blot assay. The (poly)peptides and antibodies can be bound in many different carriers and used to isolate cells specifically bound to said polypeptides. Examples of well-known carriers include glass, polystyrene, polyvinyl chloride, polypropylene, polyethylene, polycarbonate. dextran, nylon, amyloses, natural and modified celluloses, polyacrylamides. agaroses, and magnetite. The nature of the carrier can be either soluble or insoluble for the purposes of the invention.
There are many different labels and method of labeling known to those of ordinary skill in the art. Examples of the types of labels which can be used in the present invention include enzymes, radioisotopes, colloidal metals, fluorescent compounds, chemiluminescent compounds, and bioluminescent compounds. The here described diagnostic compositions are particularly useful for the detection of an activated status of the immune system, in particular to detect activation of T-cells, B-cells, NK-cells and/or monocytes.

Said diagnostic compositions may also be use for methods for detecting expression of a polynucleotide encoding TZON7 (or its homologues) by detecting the presence of mRNa coding for a TZON7 protein which comprises obtaining mRNA from a cell and contacting the mRNA so obtained with a probe comprising a nucleic acid molecule of at least 15 nucleotides capable of specifically hybridizing with a polynucleotide encoding TZON7 (or its homologues) under suitable hybridizing conditions (see also supra), detecting the presence of mRNA hybridized to the probe, and thereby detecting the expression of the TZON7 protein (or its homologues) by the cell.
Furthermore, also disclosed are methods of detecting the presence of a TZON7 protein in a sample, for example, a cell sample, which comprises obtaining a cell sample from a subject, contacting said sample with one of the aforementioned antibodies under conditions permitting binding of the antibody to the TZON7 protein, and detecting the presence of the antibody so bound, for example, using immuno assay techniques such as radio-immunoassay or enzyme-immunoassay. Furthermore, one skilled in the art may specifically detect and distinguish polypeptides which are functional TZON7 proteins from a mutated forms which have lost or altered their leukocyte (T-cell, B-cell, etc.) stimulatory activity by using an antibody which either specifically recognizes a (poly)peptide which has TZON7 activity but does not recognize an inactive form thereof or which specifically recognizes an in inactive form but not the corresponding polypeptide having TZON7 activity. The antibodies as described in the present invention may also be used in affinity chromatography for purifying the TZON7 protein or above described (poly)peptides and isolating them from various sources. Said purified proteins/(poly)peptides may be employed in the pharmaceutical compositions, uses an/or method of the present invention.

Furthermore, the indention relates to a method of diagnosing a pathological condition or susceptibility to a pathological condition in a subject related to a disorder in the immune system comprising the steps of determining the presence or amount of expression of the protein of the invention in a biological sample and diagnosing a pathological condition or a susceptibility to a pathological condition based on the presence or amount of expression of the protein.

Also disclosed is a method for diagnosing in a subject a predisposition (susceptibility) to a disorder associated with the expression of a TZON7 allele which comprises isolating DNA from victims of the disorder associated with the under- or over-expression of a TZON7 protein; digesting the isolated DNA with at least one restriction enzyme; electrophoretically separating the resulting DNA fragments on a sizing gel; contacting the resulting gel with a nucleic acid probe as described above capable of specifically hybridizing to DNA encoding a TZON7 protein and labeled with a detectable marker; detecting labeled bands on the gel which have hybridized to the labeled probe to create a band pattern specific to the DNA of victims of the disorder associated with the expression of a TZON7 protein; preparing the subject's DNA according to the above-mentioned steps to produce detectable labeled bands on a gel; and comparing the band pattern specific to the DNA of victims of the disorder associated with the expression of a TZON7 protein and the subject's DNA to determine whether the patterns are the same or different and to diagnose thereby predisposition to the disorder if the patterns are the same. The detectable markers of the present invention may be labeled with commonly employed radioactive labels, such as, for example, ³²P or ³⁵S , although other labels such as biotin or mercury as well as those described above may be employed as well. Various methods well-known the person skilled in the art may be used to label the detectable markers. For example, DNA sequences and RNA sequences may be labeled with ³²P or ³⁵S using the random primer method. Once a suitable detectable marker has been obtained, various methods well-known to the person skilled in the art may be employed for contacting the detectable marker with the sample of interest. For example. DNA-DNA, RNA-RNA and DNA-RNA hybridizations may be performed using standard procedures. Various methods for the detection of nucleic acids are well-known in the art, e.g., Southern and northern blotting, PCR. primer extension and the like. Furthermore, the mRNA, cRNA, cDNA or genomic DNA obtained from the subject may be sequenced to identify mutations which may be characteristic fingerprints of TZON7 mutations in disorders associated with the expression of TZON7 or mutated versions thereof. The present invention further comprises methods, wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, Rnase T₂ or the like or a ribozyme and, for example, electrophoretically separating and detecting the RNA fragments on PAGE as described above or in the appended examples.

TZON7 polypeptides may be used to screen for molecules that bind to TZON7 or for molecules to which TZON7 binds. The binding of TZON7 and the molecule may activate (agonist),increase, inhibit (antagonist), or decrease activity of the TZON7 or the molecule bound. Examples of such molecules include antibodies (including single-chain antibodies), oligonucleotides, proteins (e.g., receptors), or small molecules preferably, the molecule is closely related to the natural binding partner of. TZON7, e.g., a fragment of the binding partner, or a natural substrate, a "ligand", a structural or functional mimetic; see, e.g., Collgan, Current Protocols in Immunology 1(2) (1991); Chapter 5. Similarly, the molecule can be closely related to the natural binding partner(s) with which TZON7 interacts, or at least, a fragment of said binding and/or interaction partner capable of being bound by TZON7 (e.g., active site). In either case, the molecule can be rationally designed using known techniques; see also infra. (A) potential binding partner(s) of TZON7 is/are G-protein interacting molecule(s).
Preferably, the screening for these molecules involves producing appropriate cells which express TZON7, either as a secreted protein of as a protein in or on the cell membrane. Preferred cells include cells from mammals, yeast, Drosophila, or E.coli. Cells expressing TZON7 (or cell membrane(s) containing the expressed polypeptide) are then preferably contacted with a test compound potentially containing the molecule to observe binding, stimulation, or inhibition of activity of either TZON7 or the molecule. The assay may simply test binding of a candidate compound to TZON7, wherein binding is detected by a label, or in an assay involving competition with a labeled competitor. Further, the assay may test whether the candidate compound results in a signal generated by binding to TZON7. Altematively, the assay can be carried out using cell-free preparations. polypeptide/molecule affixes to a solid support, chemical libraries, or natural product mixtures. The assay may also simply comprise the steps of mixing a candidate compound with a solution containing the TZON7/molecule activity or binding to a standard.
Preferably, an ELISA assay can measure TZON7 level or activity in a sample (e.g., biological sample) using a monoclonal or polyclonal antibody. The antibody can measure TZON7 level or activity by either binding, directly or indirectly, to TZON7 or by competing with TZON7 for a substrate.
All of these above assays can be used as diagnostic or prognostic markers. The molecules discovered using these assays can be used to treat disease or to bring about a particular result in a patient (e.g., increase of immune response) by activating or inhibiting the TZON7/molecule. Moreover, the assays can discover agents which may inhibit or enhance the production of TZON7 from-suitably manipulated cells or tissues.

In summary the invention relates to a method for determining the status of the immune system comprising analyzing the presence of the polynucleotide or the protein of the invention.

Furthermore, the present invention relates to the use of the polynucleotide, the nucleic acid molecule, the vectors, peptides, polypeptides, antibodies and cells described herein as well as compounds identified in accordance with a method of the invention described herein above for the preparation of a composition for diagnosing acute and chronic diseases involving T-cell activation and associated with Th1 and Th2 immune response, It is particularly preferred that the polynucleotide encoding TZON7 (or (a) fragment(s) thereof) or the antibody as defined herein above is employed for the detection of leukocyte/lymphocyte activation.

In a further embodiment the invention relates to the use of the polynucleotides, nucleic acid molecules or antibodies of the invention for the detection of leukocyte activation as described herein above.

In a preferred embodiment said leukocyte is a B-cell, T-cell, NK-cell and/or monocyte.

These and other embodiments are disclosed and encompassed by the description and Examples of the present invention.

### BRIEF DESCRIPTION OF DRAWINGS

This disclosure may best be understood in conjunction with the accompanying drawings, incorporated herein be references, which show:
Figure 1a: Nucleotide sequence of TZON7
Figure 1b: Amino acid sequence of TZON7 cDNA
Figure 2: Translation of TZON7 cDNA sequence
Figure 3: Antigenicity blot of TZON7 amino acid sequence
Figure 4: Antigenicity blot of TZON7 amino acid sequence

A better understanding of the present invention and of is many advantages will be had the following examples, given by way of illustration.

### Example 1: Identification of a novel cDNA fragment, TZON7, that is differentially expressed in alloactivated human T cell lines

To identify novel genes induced during the early stages of T cell activation in response to allo-antigens, differential display RT-PCR (DDRT-PCR) analysis of mRNA expression was performed at time 0 and 24h after stimulation of a preconditioned human T cell line with allo-antigen. The preconditioned T cell line was prepared as follows: In conformance with institutional policies regarding human experimentation, peripheral blood lymphocytes (PBLs) were isolated from the healthy human volunteers using standard Ficoll centrifugation methods and diluted into RPMI containing 10% fetal calf serum. Isolated human PBLs (responder PBLs) were stimulated with equal numbers of irradiated (3000 rad, 13 min) stimulator PBLs from another healthy individual. Cells were co-cultured in tissue flasks at an initial concentration of 10⁶ cells/ml restimulated with stimulator cells three times in 10 day intervals prior to RNA isolation. Total RNA was isolated from cells at 0 and 24 h after last stimulation using the RNAzol B method (Tel-Test, Inc) and differential display was performed as described previously (Kojima et al., 1996). DDRT-PCR is a method which yields unbiased analysis of changes in message levels from cDNA amplified with multiple sets of primers followed by parallel 6% polyacrylamide gel electrophoresis. Briefly, 2µg of total RNA was reverse transcribed using an oligo-dT primer and 200 U MMLV reverse transrciptase (Gibco/BRL). A 40 cycle PCR amplification with a total volume of 10 µl was performed by using 1µg of cDNA, 1,25mM MgCl₂, 50 mM KCl, 10 mM Tris-HCl (pH 8,3), 2,5 nM primer, 5 µCl³⁵ S-dATP, and 0,3 U Taq polymerase. The primers for the PCR amplification were: 5'-TGCTTCAGCACTGCC -3' (SEQ ID NO:6) and -5'TTATTGTATTTGAAGTAA-3' (SEQ ID NO:7). The PCR products were stored at 4°C and separated by electrophoresis in 6% polyacrylamide-urea gels, transferred to filter paper, dried, and autoradiographed. The differentially expressed cDNA fragment was excised from the gel, eluted, reamplified, cloned into pBluescriptSK⁺ plasmid, and sequenced. Homology searches were performed using BLAST at NCBI. Alignments were performed using Geneworks 2.1.1.

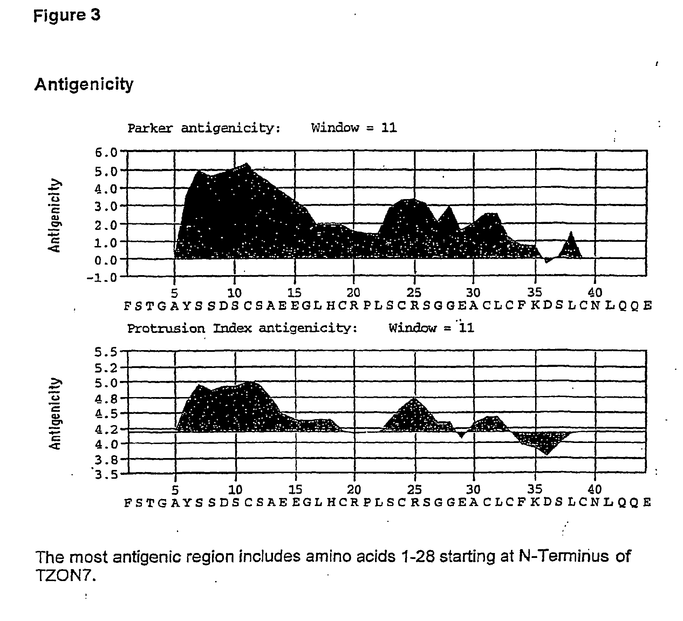

Analysis of the cDNAs showed several genes at 24 hours. One of the upregulated transcripts, TZON7, was reamplified, subcloned and 135 base pairs were sequenced. By searching GenBank, the deduced 44 amino acid sequence of TZON7 showed 18% identity to human zonadhesin molecule (GenBank accession number: U83191). Most antigenic region of the TZON7 molecule is localized between amino acid 5-14 and 22-33 as shown in Figure 3.

### Example 2: Identification and cloning of a TZON7 full-length cDNA

To obtain a full length cDNA, the 135 bp fragment obtained in Example 1 was used to screen cDNA libraries (OriGene, human peripheral blood leucocytes) and HPB-ALL (human acute lymphoblastic leukemia cell line) cDNA. The differentially expressed cDNA fragment was excised from the filter, eluted in 0.5 M ammonium acetate/1 mM EDTA, pH 8.3, and ethanol precipitated. The cDNA product was reamplified, electrophoresed in a 2% agarose gel and purified using Gene Clean kit (Quiagen). The recovered cDNA product was blunt-ended with Klenow enzyme (Gibco BRL) following standard protocols [11] and ligated into pBluescript SK⁺ vector. After labeling with α³²P[dCTP] (800 mCi/mmol, Amersham Inc.) using the random priming method [12], the cDNA fragment isolated from DDRT-PCR was used as a probe to screen the mentioned cDNA libraries. Hybridization was carried out for 24 h at 42°C in 40% formamide, 10% Dextran sulfate, 4 x SSC (1 x SSC consists of 150 mM NaCl, 15 mM sodium citrate, pH 7.0), 0.8 x Denhardt's solution (1 x Denhardt's solution contains 0.02% polyvinylpyrrolidone, 0.02% Ficoll, 0.02% bovine serum albumin), 0.5% sodium dodecyl sulfate (SDS), and 20 µg/ml salmon sperm DNA. The filters were washed twice for 20 min with 2 x SSC/10% SDS at room temperature, and for 30 min with 0.2 x SSC/10% SDS at 65 C, followed by autoradiography. From about 2 x 10⁶ recombinants, two positive clones were plaque purified, subcloned into pBluescript SK⁺ vector. PCR screening of the mentioned libraries gave similar results. Complete cDNA inserts were sequenced according to the method of Sanger [13], using a primer walking strategy starting from primers flanking the multiple cloning site of the plasmid. Sequence analysis was performed using Geneworks software system. For homology searches NCBI BLASTx and BLASTn software were used. Several clones were isolated and sequenced. Sequence analysis of one alone revealed a 822 bp cDNA (SEQ ID NO: 8) predicting a protein length of 274 amino acids (SEQ ID NO: 2).
The calculated molecular weight is 29428.02 Dalton and the estimated pl is 9.49. The amino acid composition is as follows:

| Non-polar: | No. Percent | | |
|---|---|---|---|
| | A | 34 | 12.36 |
| | V | 25 | 9.09 |
| | L | 27 | 9.82 |
| | I | 13 | 4.73 |
| | P | 12 | 4.36 |
| | M | 4 | 1.45 |
| | F | 18 | 6.55 |
| | W | 6 | 2.18 |

| Polar: | No. Percent | | |
|---|---|---|---|
| | G | 27 | 9.82 |
| | S | 25 | 9.09 |
| | T | 13 | 4.73 |
| | C | 2 | 0.73 |
| | Y | 9 | 3.27 |
| | N | 3 | 1.09 |
| | Q | 10 | 3.64 |

| Acidic: | No. Percent | | |
|---|---|---|---|
| | D | 9 | 3.27 |
| | E | 8 | 2.91 |

| Basic: | No. Percent | | |
|---|---|---|---|
| | K | 10 | 3.64 |
| | R | 13 | 4.73 |
| | H | 6 | 2.18 |

A parker antigenicity plot revealed regions with highest immunogenicity (see Figure 4):

| | |
|---|---|
| 31-44: | KTRLQSPQGFNKAG |
| 52-62: | GVPSAAIGSFP |
| 109-120: | SEWKQRAQVSA |
| 184-222: | VCGAFAGGFAAAVTTPLDVAKTRITLAKAGSSTADGYVL |

PSORT analysis shows the likelihood of the localization of the protein in (%) in the following cellular structures:

| | |
|---|---|
| 34.8% | mitochondrial |
| 26.1% | cytoplasm |
| 17.4% | endoplasmatic Reticulum |
| 13% | golgi |
| 4.3% | vaculoar |
| 4.3% | nuclear |

The translated aminoacid sequence of 274 amino acids does share very high hologies (>80% identities) with murine proteins AK015954 and AK015299. Beyond this, homologies has been found to be present in "Mitochondrial Carrier Protein Family, Pet8p' in *S*. *cerevisiae* (43% identities).

The function of the murine proteins is so far unknown. The proteins of the Pet8p family play an essential role in cellular energy metabolism. Given the sequence homology between TZON7 and "Mitochondrial Carrier Protein family Pet8p", it is likely that TZON7 could be important for growth of activated T cells and possible applied as a potential therapeutic marker or may provide novel approaches for monitoring human organ transplantation and leukemia. Means and methods to test the biological activity of the (poly)peptides of the invention, antibodies, antisense constructs and other compounds described in the general description of the invention can be performed as described in the examples of, for example, WO99/11782 and WO01/32614 which herewith are incorporated by reference.

### References

- (1): G.R. Crabtree, Contingent genetic regulatory events in T lymphocyte activation, Science 248 (1989) 355-361.
- (2): C.H. June, Signal transduction in T-cells, Curr. Opin. lmmunol. 3 (1991) 287-293
- (3): R.H. Schwartz, Costimulation of T lymphocytes: The role of CD28, CTLA-4, and B7/BB1 in Interleukin-2 production and immunotherapy, Cell 71 (1992), 1065-1068
- (4): J. Banchereau, F. Bazan, D. Blanchard, F. Briere, J. Galizzi, C. van Kooten, Y.
Liu, F. Rousset, S. Seeland, The CD40 antigen and ist ligand, Annu. Rev. lmmunol. 12 (1994) 881-992.
- (5): D.J. Lenschow, T. Walunas, J. Bluestone, CD28/B7 system of T-cell costimulation, Annu. Rev. lmmunol. 14 (1996) 233-258.
- (6): P. Linsley, J. Ledbetter, The role of the CD28 receptor during T-cell responses to antigen, Annu. Rev. lmmunol. 11 (1993) 191-212.
- (7): A. Kupfer, S.L. Swain, S.J. Singer, The specific direct interaction of helper T-cells and antigen-presenting B cells. II. Reorientation of the microtubule organizing center and reorganization of the membrane-associated cytoskeleton inside the bound helper T-cells, J. Exp. Med. 165 (1987) 1565-1580.
- (8): M.V. Parsey, G.K. Lewis, Actin polymerization and pseudopod reorganization accompany anti-CD3-induced growth arrest in Jurkat T-cells, J. Immunol. 151 (1993) 1881-1893.
- (9): N. Selliah, W.H. Brooks, T.L. Roszman, Proteolytic cleavage of -actinin by calpain in T-cells stimulated with anti-CD3 monoclonal antibody, J. Immunol. 156 (1996) 3215-3221.
- (10): R. Kojima, J. Randall, B.M. Brenner, S.R. Gullans, Osmotic stress protein 94 (Osp94): A new member of the Hsp119/SSE gene subfamily, J. Biol. Chem. 271 (1996) 12327-12332.
- (11): J. Sambrook, E.F. Fritsch, T. Maniatis, Molecular Cloning: A Laboratory Manual, Cold Spring Harbour, New York, 1989.
- (12): A.P. Feinberg, B. Vogelstein, A technique for radiolabeling DNA restriction endonuclease fragments to high specific activity, Anal. Biochem. 132 (1983) 6-13.
- (13): F. Sanger, S. Nicklen, A.R. Coulson, DNA sequencing with chain-terminating inhibitors, Proc. Natl. Acad. Sci. USA 74 (1977) 5463-5467.

## Claims

1. An in vitro method for determining the status of an immune response comprising
(a) determining the presence of a polynucleotide encoding a TZON7 polypeptide in a sample, wherein said polynucleotide comprises a nucleic acid molecule selected from the group consisting of
(i) DNA sequences encoding the amino acid sequence depicted in any one of SEQ ID NOs: 9 to 13;
(ii) the DNA sequence depicted in SEQ ID NO: 8;
(iii) DNA sequences the complementary strand of which hybridizes with and which is at least 90% identical to the polynucleotide as defined in any one of (i) to (ii); and
(iv) DNA sequences the nucleotide sequence of which is degenerated to the nucleotide sequence of a DNA sequence of any one of (i) to (iii); wherein
(b) an increased expression of said polynucleotide is indicative for an activated status of an immune response.

2. An in vitro method for determining the status of an immune response comprising
(a) determining the presence or amount of expression of a protein encoded by a polynucleotide as defined in claim 1 in a sample; wherein
(b) an increased expression of said polynucleotide is indicative for an activated status of an immune response.

3. Use of a polynucleotide as defined in claim 1, a nucleic acid molecule of at least 15 nucleotides in length hybridizing specifically with said polynucleotide or with a complementary strand thereof, a vector comprising a polynucleotide as defined in claim 1 or said nucleic acid molecule, optionally linked to regulatory sequences allowing for the transcription and/or expression of said polynucleotide or nucleic acid molecule, a protein as defined in claim 2 or an antibody specifically recognizing said protein for the preparation of a composition for diagnosing acute and chronic diseases, involving T cell activation and Th1 and Th2 immune response.

4. Use of a polynucleotide or an antibody as defined in claim 3 for the detection of leukocyte/lymphocyte activation.

5. Use of claim 4, wherein said leukocyte/lymphocyte is a B cell, T cell, NK cell and/or monocyte.

## Patentansprüche

1. In vitro Verfahren zum Bestimmen des Status einer Immunantwort umfassend
(a) Bestimmen des Vorhandenseins eines Polynukleotids, das für ein TZON7-Polypeptid kodiert, in einer Probe, wobei das Polynukleotid ein Nukleinsäuremolekül umfasst, das ausgewählt ist aus der Gruppe bestehend aus
(i) DNA-Sequenzen, die für die in einer der SEQ ID NOs: 9 bis 13 dargestellten Aminosäuresequenz kodieren;
(ii) die in SEQ ID NO: 8 dargestellte DNA-Sequenz;
(iii) DNA-Sequenzen, deren komplementärer Strang mit dem Polynukleotid, wie in einem von (i) bis (ii) definiert, hybridisiert und mindestens 90 % identisch dazu ist; und
(iv) DNA-Sequenzen, deren Nukleotidsequenz zu der Nukleotidsequenz einer DNA-Sequenz nach einem von (i) bis (iii) degeneriert ist; wobei
(b) eine erhöhte Expression des Polynukleotids indikativ ist für einen aktivierten Status einer Immunantwort.

2. In vitro Verfahren zum Bestimmen des Status einer Immunantwort umfassend
(a) Bestimmen des Vorhandenseins oder der Menge an Expression eines Proteins, das von einem Polynukleotid, wie in Anspruch 1 definiert, kodiert wird, in einer Probe; wobei
(b) eine erhöhte Expression des Polynukleotids indikativ ist für einen aktivierten Status einer Immunantwort.

3. Verwendung eines Polynukleotids wie in Anspruch 1 definiert, eines Nukleinsäuremoleküls von mindestens 15 Nukleotiden Länge, das spezifisch mit dem Polynukleotid oder mit einem komplementären Strang davon hybridisiert, ein Vektor umfassend ein Polynukleotid wie in Anspruch 1 definiert, oder das Nukleinsäuremolekül, gegebenenfalls verknüpft mit regulatorischen Sequenzen, welche die Transkription und/oder Expression des Polynukleotids oder des Nukleinsäuremoleküls erlauben, ein Protein wie in Anspruch 2 oder ein Antikörper, der spezifisch dieses Protein erkennt, für die Herstellung einer Zusammensetzung zum Diagnostizieren akuter und chronischer Erkrankungen, die T-Zellaktivierung und Th1- und Th2-Immunantwort mit sich bringen.

4. Verwendung eines Polynukleotids oder eines Antikörpers wie in Anspruch 3 definiert, für den Nachweis von Leukozyten-/Lymphozytenaktivierung.

5. Verwendung von Anspruch 4, wobei der Leukozyt/Lymphozyt eine B-Zelle, T-Zelle, NK-Zelle und/oder ein Monozyt ist.

## Revendications

1. Procédé in vitro de détermination de l'état d'une réponse immunitaire comprenant:
(a) la détermination de la présence d'un polynucléotide codant pour un polypeptide TZON7 dans un échantillon, dans lequel ledit polynucléotide est constitué d'une molécule d'acide nucléique choisie dans le groupe constitué par
(i) des séquences d'ADN codant pour la séquence d'acides aminés représentée par l'une quelconque de SEQ ID NOs: 9 à 13;
(ii) la séquence d'ADN représentée par SEQ ID NO: 8;
(iii) des séquences d'ADN dont le brin complémentaire s'hybride et qui est au moins à 90 % identique au polynucléotide tel que défini dans l'un quelconque de (i) à (ii); et
(iv) des séquences d'ADN dont la séquence nucléotidique est dégénérée par rapport à la séquence nucléotidique d'une séquence d'ADN de l'un quelconque de (i) à (iii);
dans lequel
(b) une expression accrue dudit polynucléotide est indicatrice d'un état activé d'une réponse immunitaire.

2. Procédé in vitro de détermination de l'état d'une réponse immunitaire comprenant:
(a) la détermination de la présence ou de la quantité d'expression d'une protéine codée par un polynucléotide tel que défini dans la revendication 1 dans un échantillon; dans lequel
(b) une expression accrue dudit polynucléotide est indicatrice d'un état activé d'une réponse immunitaire.

3. Utilisation d'un polynucléotide tel que défini dans la revendication 1, d'une molécule d'acide nucléique d'au moins 15 nucléotides en longueur s'hybridant spécifiquement au dit polynucléotide ou à un brin complémentaire de celui-ci, d'un vecteur comprenant un polynucléotide tel que défini dans la revendication 1 ou ladite molécule d'acide nucléique, éventuellement lié à des séquences régulatrices permettant la transcription et/ou l'expression dudit polynucléotide ou de ladite molécule d'acide nucléique, d'une protéine telle que définie dans la revendication 2 ou d'un anticorps reconnaissant spécifiquement ladite protéine pour la préparation d'une composition destinée au diagnostic de maladies aiguës et chroniques, impliquant l'activation des lymphocytes T et une réponse immunitaire de type Th1 et Th2.

4. Utilisation d'un polynucléotide ou d'un anticorps tel que défini dans la revendication 3 pour la détection d'une activation de leucocytes/lymphocytes.

5. Utilisation selon la revendication 4, dans laquelle ledit leucocyte/lymphocyte est un lymphocyte B, un lymphocyte T, une cellule NK et/ou un monocyte.
